# EUROPEAN PATENT APPLICATION

(11) **EP 4 530 380 A1**
(43) Date of publication of application: **02.04.2025**
(21) Application number: 23811777.4
(22) Date of filing: 22.05.2023
(51) Int. Cl.: D01D 5/06, A61L 27/18, A61L 27/46, D01F 6/62, D01F 6/92

(54) **METHOD FOR CONTINUOUSLY PRODUCING BIODEGRADABLE FIBER MATERIAL CONTAINING INORGANIC FILLER PARTICLES USING WET SPINNING, AND COTTON-LIKE BONE REGENERATION MATERIAL PRODUCED WITH SAID METHOD**

(30) Priority: 23.05.2022 US 202263344913 P
(71) Applicant: Nagoya Institute Of Technology, Nagoya-shi, Aichi 466-8555 (JP); Orthorebirth Co., Ltd., Yokohama-shi, Kanagawa 224-0033 (JP)
(72) Inventor: KASUGA Toshihiro, Nagoya-shi, Aichi 466-8555 (JP); MATSUBARA Takashi, Nagoya-shi, Aichi 466-8555 (JP); KUMANO Masahiro, Yokohama-shi, Kanagawa 224-0033 (JP)
(74) Representative: Weihs, Bruno Konrad
(86) International application number: PCT/JP2023/018963
(87) International publication number: WO 2023/228905

(57) **Abstract**

A method for producing biodegradable fibers containing inorganic filler particles by wet spinning, prepared by dissolving a mixture of inorganic filler particles and biodegradable resin in a weight ratio of 50-80:50-20 in a good solvent and stirring the The solution is filled into a syringe of a wet spinning machine and extruded downward from the discharge port of the injection needle into a collector container. The collector vessel is filled with a first poor solvent having a dissolution parameter value with a small deviation from the good solvent and a second poor solvent having a specific gravity greater than the first poor solvent and a dissolution parameter value with a large deviation from the good solvent, in two layers, top and bottom. The spinning solution extruded from the syringe outlet enters the first poor solvent in the collector container by its own weight in fibrous form, and then continuously enters the second poor solvent filled below the first poor solvent. The fibrous spinning solution injected into the second poor solvent becomes continuous long fibers and is deposited floating on the bottom of the collector container to form a cotton shape.

## Description

### [Technical Field]

Present invention relates to a method for continuously producing a biodegradable fibrous material containing inorganic filler particles using a wet spinning method, and a cotton-wool like bone regeneration material produced by the method. The invention further relates to a method for producing nonwoven fabrics comprising biodegradable fibers containing inorganic filler particles using a wet spinning method.

### [Background Technology]

In the area of bone regeneration therapy, polylactic acid is used as a matrix polymer that is mixed with calcium salt particles (beta-tricalcium phosphate, silicon-eluting calcium carbonate, hydroxyapatite, etc.) to produce a fibrous material as a bone regeneration material using electrospinning method(ES). While bone regeneration materials are generally used in block or granule form, bone regeneration materials made of biodegradable fibers produced by this method have excellent formability during surgery and have excellent features that resolve concerns about migration and/or dropout from the target site. Inventors of present invention have succeeded in producing a cotton-wool like structure by receiving biodegradable fibers emitted from a nozzle using ES in a collector container filled with ethanol and collecting and drying the fibers deposited in the ethanol solution (US8853298). Cotton-wool like bone regeneration materials are clinically superior because they can be easily adapted to any geometry of a bone defect during surgery.

PLGA is an excellent biodegradable resin that is more bioabsorbable than polylactic acid and its safety is approved by FDA. PLGA is synthesized by copolymerization of lactic acid and glycolic acid, and its biodegradability can be controlled by adjusting the ratio of lactic acid and glycolic acid. In the cases of PLGA (85:15), which is 85% lactic acid: 15% glycolic acid, and PLGA (75:25), which is 75% lactic acid: 25% glycolic acid, PLGA (75:25) is more degradable. On the other hand, lactic acid of polylactic acid has a crystalline L-isomer and an amorphous D-isomer, which is an optical isomer of L-isomer. PDLLA containing D-isomer is more difficult to be crystallized and more easily degraded than PLLA, which contains only L-isomer without D-isomer. Therefore, it is possible to synthesize PDLLGA having much higher degradability than PLLGA that does not contain D-isomer by copolymerizing PDLLA containing D-isomer and PGA.

ES is an excellent method for producing a bone regeneration material comprising biodegradable fibers, because inorganic filler particles, which are bone-forming factors, can be contained in the spinning solution and made into fibers. However, ES requires special equipment for this purpose and its production cost is high. In addition, in order to electro-spin a fiber containing a large amount of inorganic filler particles, the inorganic filler particles must be uniformly dispersed in the spinning solution. Therefore, in order to electro-spin a fiber containing a large amount of inorganic filler particles, it is necessary to undergo a process such as kneading process in the production of spinning solution, which further increases the production cost. In addition, since ES produces fibers by having spinning solution fly in an electric field through a nozzle by applying a high voltage, it is difficult to maintain the fiber shape when PDLLGA having low molecular weight is used. As a result, it is difficult to electro-spin a biodegradable fiber using PDLLGA as a matrix polymer.

In addition to cotton-wool like bone regeneration materials, inventors of the present invention have developed a cell culture substrate using non-woven fabrics produced by using ES (patent numbers 6602999 and 6639035). It has been found that a non-woven fabric produced by using ES has a good cell adhesion property because inorganic particles contained in the fiber are exposed on the surface of the fibers, and because of that the nonwoven fabric can be used as an excellent three-dimensional cell culture substrate. However, the same problem that is associated with the use of ES exists.

### [Prior art references]

### [Patent documents]

U.S. Patent No. 8853298
U.S. Patent No. 6602999

### Summary of Invention

### [Problem to be solved by the invention]

As a method for spinning biodegradable fibers containing inorganic filler particles, wet spinning method can be used instead of ES. A spinning solution is prepared by feeding inorganic filler particles into a solution of biodegradable resin dissolved in a good solvent and stirring to disperse the filler particles in the solution, and the spinning solution prepared in this manner is fed in a syringe and extruded through a nozzle to inject the spinning solution into a collector container that is filled with poor solvent in fibrous form. By this method, composite biodegradable fibers containing inorganic filler particles can be produced in a poor solvent.

In the wet spinning method, the spinning solution excluded from the nozzle in fibrous form enters into the poor solvent, and the good solvent contained in the spinning solution is desorbed into the poor solvent. If the speed at which the good solvent penetrates into the poor solvent is too slow, it is difficult for the spinning solution excluded from the nozzle to make fibers in the poor solvent. Conversely, if the rate at which the good solvent penetrates into the poor solvent is too fast, the spinning solution will clog at the nozzle. In particular, if the spinning solution contains a large amount of inorganic particles, clogging is more likely to occur at the place where the spinning solution is injected into the poor solvent through the narrow outlet of the nozzle.

### [Means to solve the problem]

In order to solve above problem and achieve efficient wet spinning, inventors of the present invention, after intensive study, reached the idea of filling two kinds of poor solvents each having a different solubility, in two layers, top and bottom, in a collector vessel of a wet spinning apparatus.
That is, spinning solution prepared by feeding a sample (inorganic particles and biodegradable resin) with an approximately equal amount of good solvent is extruded from a nozzle outlet of a wet spinning apparatus into a collector vessel. The spinning solution extruded from the nozzle outlet is injected in a fibrous form into the first poor solvent, difference of dissolution parameter of the first poor solvent from that of the good solvent is relatively small, and then the spinning solution injected in a fibrous form is entered into the second poor solvent, difference of dissolution parameter of the second poor solvent from that of the good solvent is larger than that of the first poor solvent, so that a fiber containing a large amount of inorganic particles can be efficiently produced.

Based on above finding, inventors of the present invention reached a method for producing biodegradable fibers containing inorganic filler particles using a wet spinning process, the method comprising:
putting a mixture of inorganic filler particles and biodegradable resin in a weight ratio of 50-80:50-20 into a mixing vessel;
charging a predetermined amount of a good solvent to the mixture in the mixing vessel so that the biodegradable resin of the mixture is dissolved by the good solvent, and stirring the dissolved mixture to prepare a spinning solution containing inorganic filler particles dispersed in the solution;
filling a syringe of a wet spinning apparatus with the spinning solution thus prepared;
excluding the spinning solution filled in the syringe through an outlet of an injection needle having a predetermined diameter at a predetermined extrusion rate in a downward direction so that the spinning solution is injected into a cylindrical collector container having a predetermined height, wherein the collector container is filled with a first poor solvent and a second poor solvent in two layers, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance,
the spinning solution extruded through the outlet of the nozzle is entered into the first poor solvent filled in the collector container in a fibrous form by its own weight, a surface of the spinning solution entered into the first poor solvent in the fibrous form is solidified by interdiffusion of desorption of the good solvent and penetration of the first poor solvent;
the spinning solution in the fibrous form having a solidified surface is then continuously entered into the second poor solvent below the first poor solvent in the collector vessel by its own weight;
the spinning solution entered into the second poor solvent in the fibrous form is further solidified to produce a solidified fiber by proceeding interdiffusion of desorption of the good solvent and penetration of the second poor solvent into an interior of the fibrous spinning solution, and the solidified fiber becomes a long continuous fiber without adhering to each other, and floating deposited at a bottom of the collector vessel; and
collecting the fibers deposited at the bottom of the collector container from the collector container and drying the collected fibers to produce a biodegradable fiber containing inorganic filler particles.

Inventors of present invention further developed a cotton-wool like bone regenerating material produced by using a wet spinning method, the cotton-wool like bone regenerating material is produced by the steps comprising:
putting a mixture of inorganic filler particles and biodegradable resin in a weight ratio of 50-80:50-20 into a mixing vessel;
charging a predetermined amount of a good solvent into the mixing vessel so that the biodegradable resin of the mixture is dissolved by the good solvent, and stirring the dissolved solution to prepare a spinning solution in which the inorganic filler particles are dispersed in the solution;
filling the spinning solution thus prepared in a syringe of a wet spinning apparatus;
excluding the spinning solution filled in the syringe through an outlet of an injection needle having a predetermined diameter at a predetermined extrusion rate in a vertical downward direction so that the spinning solution is injected into a cylindrical collector container having a predetermined height, wherein the collector container is filled with a first poor solvent and a second poor solvent in two layers, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance,
the spinning solution extruded through the outlet of the nozzle is entered into the first poor solvent in the collector container by its own weight in a fibrous form, a surface of the spinning solution entered into the first poor solvent in the fibrous form is solidified by interdiffusion of the desorption of the good solvent and the penetration of the first poor solvent;
the spinning solution in the fibrous form having a solidified surface is then continuously entered into the second poor solvent below the first poor solvent in the collector vessel by its own weight;
the spinning solution entered into the second poor solvent in the fibrous form is further solidified to produce a solidified fiber by allowing the interdiffusion of desorption of good solvent and penetration of second poor solvent into the interior of the spinning solution to proceed, and a long continuous fiber is produced by the solidified fibers without adhering each other and floating deposited at the bottom of the collector vessel;
collecting the fiber deposited at the bottom of the collector container from the collector container and dried.

Inventors of the present invention further developed a wet spinning apparatus for use in an improved wet spinning process, the apparatus comprising:
a syringe for injecting a spinning solution, the spinning solution is prepared by putting inorganic filler particles and biodegradable resin in a mixing vessel in a weight ratio of 50-80:50-20 into a mixing vessel and dissolving the biodegradable resin by using a good solvent and stirring the dissolved solution,
a nozzle connected to an end of the syringe having a discharge port at a tip end thereof,
a cylindrical collector container having a predetermined height,
the collector container comprises:
   an injection section for injecting and passing the spinning solution extruded from the nozzle outlet into a first poor solvent,
   a fiber solidification section for solidifying the spinning solution that has passed through the injection section into a second poor solvent, the fiber solidification section is provided adjacent below the injection section, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance,
   a fiber collection section adjacent below the fiber solidification section for collecting the fiber that has passed through the fiber solidification section and floating deposited at the bottom of the collector container in a cotton-wool like form,
   the fiber solidification section and the fiber collection section are separably connected each other using a clamp, and the fiber collection section can be separated from the fiber solidification section after the solidified fibers are deposited at the bottom of the collector container.

Preferably, the amount of good solvent to be added to the mixture of the inorganic filler particles and biodegradable resin is adjusted to be an amount that allows the inorganic filler particles to diffuse uniformly in the syringe without settling down in the solution and the spinning solution is made into a fiber in the poor solvent by extruding the spinning solution from the syringe needle into the poor solvent.

Preferably, the particle size of the *β*-TCP particles is 0.4-5 *µ*m.

Preferably, the spinning solution is prepared by mixing *β*-TCP particles having a particle size of 3-5 *µ*m and PDLLGA resin in a weight ratio of 50-80:50-20 and dissolving the mixture of *β*-TCP particles and PDLLGA resin by adding acetone to the mixture in a weight ratio of 0.7-1.3 : 1 (acetone : mixture).

Preferably, the spinning solution is prepared by mixing *β*-TCP particles having a particle size of 0.3-0.5 *µ*m and PDLLGA resin in a weight ratio of 50-80:50-20 and dissolving the mixture of *β*-TCP particles and PDLLGA resin by adding acetone to the mixture in a weight ratio of 1.15-1.40 : 1 (acetone : mixture).

Preferably, the position of the interface between the first poor solvent and the second poor solvent in the collector container is adjusted such that the interface comes close to the nozzle discharge port. If the distance over which the spinning solution injected in fibrous form from the discharge outlet of the injection needle passes through the first poor solvent, which has a small difference in dissolution parameters from that of the good solvent, is long, the fibers injected from the nozzle outlet are easily torn off in the middle. Therefore, the height position of the interface between the first and second poor solvents is close to the nozzle outlet so that the fibers can be deposited as a single continuous fiber at the bottom of the collector container.

Preferably, inorganic filler particles are calcium phosphate particles, more preferably *β*-TCP particles. Furthermore, *β*-TCP particle containing silver is useful because of its antimicrobial properties.

Preferably, the tubular collector vessel of the wet spinning apparatus has a height of 50 to 100 cm and is divided into an injection section, a fiber solidification section and a fiber collection section from the top to the bottom. The spinning solution extruded in fibrous form from the nozzle outlet passes by its own weight through the first poor solvent in the injection section, then is entered into the second poor solvent in the fiber solidification section passing through the fiber solidification section, and then is introduced into the fiber collection section, which is also filled with the second poor solvent, and is deposited in a cotton-wool like floating state at the bottom of the collector vessel. The cottony fibers deposited at the bottom of the collector vessel can be removed from the collector vessel by separating the fiber collection section of the collector vessel from the fiber solidification section and collected and dried to obtain cotton-wool like bone regeneration material.

Preferably, acetone is used as the good solvent, ethanol as the first poor solvent, and water as the second poor solvent.

Preferably, acetone is used as the good solvent, the first poor solvent is a mixture of acetone and water, and the second poor solvent is water.

Preferably, chloroform is used as the good solvent, ethanol as the first poor solvent, and water as the second poor solvent.

Preferably, the second poor solvent is pure water. If any of the solvents (good solvent, first poor solvent, second poor solvent) contain chlorine, there is a possibility that AgCl will be formed by reacting with the silver contained in *β*-TCP. Therefore, it is preferable that the solvents are chlorine-free.

Preferably, the biodegradable fiber contains 50-80% inorganic filler particles by weight, more preferably 60-70% by weight. In the wet spinning method, a spinning solution containing a large amount of inorganic filler particles can be easily prepared because fiber is produced by preparing a spinning solution by mixing the resin and filler particles and dissolving them in an organic solvent and then extruding the spinning solution from a syringe. Contrary to WS, ES uses a slurry with low viscosity during spinning, so that the dispersibility of inorganic filler particles must be greatly enhanced beforehand, and a special process (ex. kneading) is required to uniformly disperse a large amount of filler particles in the solution. Wet spinning does not require such a special process because it can use a slurry with higher viscosity than that of ES. This is because the polymer solution that fills the space between the particles becomes less fluid, preventing occurrence of agglomeration.

Preferably, calcium phosphate particles are used as inorganic filler particles, and more preferably, *β*-TCP particles are used. The biodegradable resin is decomposed in contact with body fluids, eluting *β*-TCP particles, which in turn elute calcium and phosphorus ions, promoting bone formation through bone resorption replacement.

Preferably, as the *β*-TCP particles, *β*-TCP particles containing silver ions are used. As the *β*-TCP particles elute from the biodegradable fiber, the silver ions contained in the *β*-TCP particles elute and exhibit antimicrobial properties.

Preferably, injection needle of the nozzle of the wet spinning apparatus uses 27G (inner diameter 0.19 ± 0.02 mm, outer diameter 0.41 ± 0.01 mm) or 22G (inner diameter 0.41 ± 0.03 mm, outer diameter 0.72 ± 0.02 mm), and outer diameter of the fiber produced by the apparatus is 80 to 200 *µ*m. More preferably, 27G needle is used for the nozzle of the wet spinning apparatus, and the outer diameter of the fiber thus produced is 100 to 150 *µ*m. In a separate experiment, the inventors found that when 22G is used for the injection needle, fibers are spun smoothly with resin concentration 16.7-20. 5% by weight and an extrusion speed of 3 ml/h. However, when the resin concentration is 15.3% by weight, the viscosity is low and inorganic particles easily settle down in the syringe, resulting in production of heterogeneous fibers. When resin concentration is 26.5% by weight, the fibers cannot be easily extruded due to high viscosity. Fibers extruded using 22G are thick and stiff. On the other hand, when a nozzle with a diameter of 27G was used, the range of appropriate resin concentration became narrower, but the fiber could be spun well at a resin concentration of 20wt% in the spinning solution and an extrusion speed of 3ml/h. The diameter of the fiber was finer than when 22G was used, and the fiber was less stiff and more excellent as a product. Unlike ES method, the wet spinning method discharges the spinning solution through a nozzle by simply pushing the spinning solution out. The resin concentration of the spinning solution can be set relatively freely according to the discharge speed and desired fiber thickness However, the appropriate resin concentration of the spinning solution depends on diameter of the nozzle.

Preferably, a metal or plastic net is submerged at the bottom of the fiber collection section of the collector container. Fibers produced by wet-spinning are deposited on the bottom. When the net is pulled up, the fibers are still soft and take on a nonwoven or cotton shape. If the fibers are dipped into water, the fibers are solidified without changing its form.

Preferably, a take-up apparatus is placed at the bottom of the collector container, and the take-up apparatus winds up the fibers that are deposited through the second poor solvent. By increasing the winding speed of the take-up apparatus, a nonwoven fabric with aligned fiber direction can be obtained.

### [Advantage of the Invention]

In the present invention, amount of sample (inorganic particles and biodegradable resin) and good solvent are adjusted such that the particles of the sample are diffused uniformly without sinking in the good solvent and the spinning solution is extruded from the outlet to become a fiber in the poor solvent. Therefore, the wet-spinning apparatus can be used to spin biodegradable on a commercial basis.

In the wet spinning method of the present invention, the spinning solution extruded in fiber form from the nozzle outlet into the poor solvent contains a large amount of inorganic particles (specific gravity of *β*-TCP is 3.14 g/cm3) in a resin solution (specific gravity of polylactic acid is 1.24). The fibers sink downward and passes the first poor solvent and the second poor solvent (specific gravity of ethanol is around 0.8, specific gravity of water is 1) due to its own weight and are gradually converted into fibers during the sinking process and deposited at the bottom of the collector container so that the deposited fibers can be collected from the container.

Since the second poor solvent filled in the fiber solidification section of the collector vessel has a greater specific gravity than the first poor solvent filled in the injection section that is above the fiber solidification section, the difference in specific gravity causes the first poor solvent to float on the second poor solvent to form two layers. Therefore, by injecting the spinning solution into the first poor solvent from the outlet of the apparatus, the spinning solution injected in fibrous form sequentially passes through the first poor solvent and the second poor solvent, each having a different dissolution parameter, due to its own weight. The spinning solution is gradually solidified to form a fiber during that process. Provided, however, that when ethanol is used as the first poor solvent and water is used as the second poor solvent, ethanol and water become mixed together if the two are stirred, by which hydrophilic groups of ethanol is coordinated by water molecule since ethanol is hydrophilic. Therefore, it is preferable to pour ethanol slowly on top of water to create a state of separation, utilizing the difference in specific gravity so that water is not allowed to coordinate around the hydrophilic groups of ethanol.

In a preferred embodiment of the invention, because the second poor solvent (e.g. water) used for solidifying the spinning solution has a greater specific gravity than the good solvent (e.g. acetone), the good solvent (e.g., acetone) desorbed from the fibers immediately floats upward without drifting near the fibers and does not remain in the vicinity of the solidified fibers after being desorbed from the fibers. As a result, because the good solvent is prevented from dissolving the fibers that have been solidified and accumulated at the bottom of the collector container, the deposited fibers do not adhere to each other and can be collected from the collector container in a cotton-wool like form.

By selecting a biodegradable resin having high solubility in solvents, a non-chlorinated solvent (e.g., acetone) can be used as a good solvent for the preparation of the spinning solution without a need to use a chlorinated solvent having high solubility (e.g., chloroform).

Biodegradable fibers produced by the wet spinning method of an embodiment of this invention have long fiber lengths and can be deposited and recovered in almost one continuous fiber in the form of cotton-wool like material. Compared to a cotton-wool like material produced by ES, the cotton-wool like bone regeneration material thus deposited and recovered from a collector container has a larger fiber-to-fiber spacing and a larger microenvironment for cells to penetrate between the fibers and adhere to the fibers to proliferate thereon.

Biodegradable fibers produced by the wet spinning method of the present invention have fewer pores on fiber surface, a denser cross-sectional structure, and better shape retention than the fibers produced by ES.

The biodegradable fibers produced by the wet spinning method of the present invention have a flat cross-sectional shape and excellent cell adhesion property.

Wet spinning method is different from ES in that the spinning solution is extruded from the syringe to the outlet by applying physical force. Because of that, there is a high degree of freedom regarding the content of filler particles in the spinning solution. By containing calcium phosphate at 50% by weight, more preferably 60% by weight, and even more preferably 70% by weight, the particles form an uneven structure on the fiber surface. The fiber surface having an uneven structure is suitable for cell adhesion.

After implantation in a body, PDLLGA is dissolved in the body and creates an acidic environment by locally lowering the pH level. As a result, *β*-TCP is dissolved in an acidic environment, releasing a small amount of calcium ions and phosphate ions to promote bone formation.

When *β*-TCP particles containing silver ions are included as filler in the biodegradable fiber produced by the wet spinning method of the present invention, the biodegradable resin is dissolved in the body and pH decreases, and the *β*-TCP filler is dissolved in an acidic environment, resulting in the elution of silver ions contained in the *β*-TCP particles and exhibiting antibacterial property. This enables the combination of PDLLGA fibers produced by the wet spinning method and silver ion-containing *β*-TCP particles to achieve the antimicrobial property in the late postoperative period after implantation of the bone regeneration material in the body.

In the wet spinning method of the present invention, acetone used as an organic solvent does not contain chlorine, so it does not produce silver chloride when it comes into contact with silver. As a result, the Ag ions contained in the *β*-TCP particles do not produce AgCl, but stay as Ag ions so that antimicrobial properties of Ag ions is exhibited. In addition, AgCl does not form and turn black when exposed to light.

Biodegradable fibers produced by the wet spinning method of the present invention exhibit excellent cell adhesion when administered to the human body because the inorganic particles contained in the fiber are exposed on the fiber surface without being covered by resin, forming an uneven shape.

### [Brief Description of the Drawings].

Figure 1 shows a conceptual diagram of the wet spinning apparatus of present invention.
Figure 2 is a SEM photograph showing the configuration of the biodegradable fiber produced in an embodiment of the wet spinning method of present invention (acetone as good solvent, ethanol as the first poor solvent, and water as the second poor solvent).
Figure 3 is a SEM photograph showing the uneven surface of the biodegradable fiber produced by the wet spinning method of present invention (acetone as good solvent, ethanol as the first poor solvent, and water as the second poor solvent).
Figure 4 shows a conceptual diagram of the distance of Hansen solubility parameter (HSP distance) between each solvent when acetone is used as the good solvent, ethanol as the first poor solvent, and water as the second poor solvent.

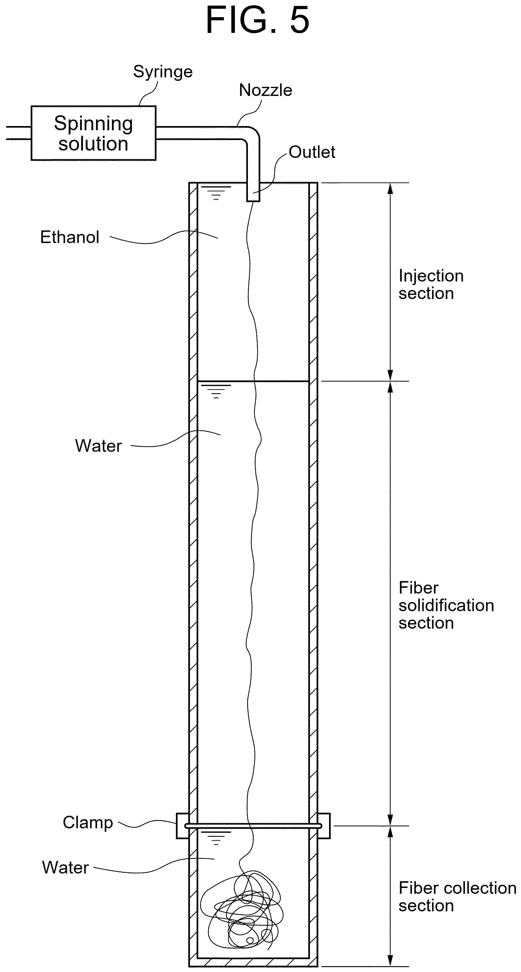

Figure 5 shows a conceptual diagram of the wet spinning apparatus used in the wet spinning method of present invention (acetone as a good solvent, ethanol as the first poor solvent, and water as the second poor solvent).

### [Embodiments for carrying out the invention]

### [Definition]

Hereinafter, embodiments of the present invention will be explained in detail with reference to the drawings.

### Definition.

### <PLLGA Resin>

In the present invention, PLLGA resin refers to PLGA resin synthesized by copolymerization of lactic acid containing only L-isomer and glycolic acid. PLLGA (85:15) refers to a polymerization ratio of 85:15 of PLLA to PGA. PLLGA (75:25) refers to a polymerization ratio of 75:25 of PLLA to PGA. Degradability of PLLGA can be enhanced by increasing the ratio of PGA. In order to dissolve PLLGA in a solvent, chlorinated solvent such as chloroform needs to be used.

### <PDLLGA resin>

In the present invention, PDLLGA resin refers to PLGA resin synthesized by copolymerization of lactic acid containing D- and L-isomers and glycolic acid. PLA includes poly(L-lactic acid) (PLLA), which contains only L-isomer, and poly(D-lactic acid) (PDLLA), which contains both L-isomer and D-isomer. PDLLGA(75:25) refers to a copolymer of PDLLA and PGA in polymerization ratio of 75:25. PDLLGA which is a copolymer of PDLLA and PGA has particularly high flexibility among PLGA. Degradability of PDLLGA can be controlled by changing polymerization ratio of PDLLA and PGA. It is difficult to specifically determine amount of D-isomer. In the present invention, amount of D-isomer in PDLLGA resin is an amount that is sufficient to make the resin degradable and dissolvable in acetone.

### [Wet spinning method]

In the present invention, wet spinning method refers to a method to solidify into fiber form by interdiffusion of desorption of an organic solvents, called good solvents, and penetration of a poor solvent. Choice of good solvent and poor solvent affects the speed of polymer solidification and the interdiffusion of solvents, and the balance of the speed of the interdiffusion determines the form of the fibers. In an embodiment of the wet spinning method, the conditions are set and improved for fibering PDLLGA resin containing calcium phosphate particles to form a cotton-wool like structure.

### <Good solvent>

In the present invention, good solvent refers to a solvent used to dissolve a mixture of biodegradable resin and calcium phosphate particles to prepare a spinning solution. Chlorinated organic solvent such as chloroform have excellent power to dissolve the resin, but are toxic. Acetone is inferior to chloroform in terms of solving power, but it does not contain chlorine and is safe for living organisms. The PDLLGA resin used in the present invention does not require the use of chlorinated organic solvent such as chloroform. It can use acetone, which is a safe non-chlorinated solvent.

In the present invention, in preparing the spinning solution, when the particle size of inorganic filler particles is about 4 *µ*m, it is preferable that the amount of good solvent is adjusted such that weight ratio of good solvent to the mixture of inorganic filler particles and biodegradable resin is 1:0.7-1.3. More preferably, weight ratio of good solvent to the mixture is approximately 1:1.
In the experiments conducted by the inventors of the present invention, if the weight ratio of good solvent (acetone) to the sample (mixture of inorganic filler particles and biodegradable resin) 1 is greater than 1.3, the particles tend to sink in the syringe and the amount of dispersed inorganic particles (β -TCP) in the early and late stages of injection changes, resulting in producing a heterogeneous product. Conversely, if the weight ratio of good solvent (acetone) to the sample 1 is less than 0.7, it becomes difficult to extrude the spinning solution from the nozzle outlet. When the amount of good solvent (acetone) to the sample (mixture of *β*-TCP and PDLLGA resin) is within the range of 1:0.7-1.3 by weight, the spinning solution can be extruded from the discharge port and made into fiber in the poor solvent.

When particle diameter of inorganic filler particles is about 0.3 to 0.5 *µ*m, it is preferable that amount of good solvent is adjusted such that weight ratio of good solvent to the mixture of inorganic filler particles and biodegradable resin is 1:1.15-1.40. According to experiments conducted by the inventors of the present invention using *β*-TCP particles having a particle size of 0.4 *µ*m, it was difficult to extrude the spinning solution out of the syringe when weight ratio of acetone to sample 1 was 1 : 1. 1. Conversely, when weight ratio of acetone to sample 1 was 1 : 1.45, the *β*-TCP particles settled down and separated in the solution over time.

### <Poor solvent>

In the present invention, poor solvent refers to the solvent that does not dissolve biodegradable resin in the coagulation bath solution. Academically, in a particular substance-solvent system, a solvent is called as a poor solvent when the solute-solvent interaction (free energy) is less than the arithmetic mean of the solute-solute and solvent-solvent interactions. Poor solvent used in the method of present invention is selected using dissolution parameter as an indicator, considering the balance between the organic solvent and the poor solvent. In the present invention, when PDLLGA is used as the biodegradable resin, ethanol or water, to both of which PDLLGA is insoluble, can be suitably used as a poor solvent.

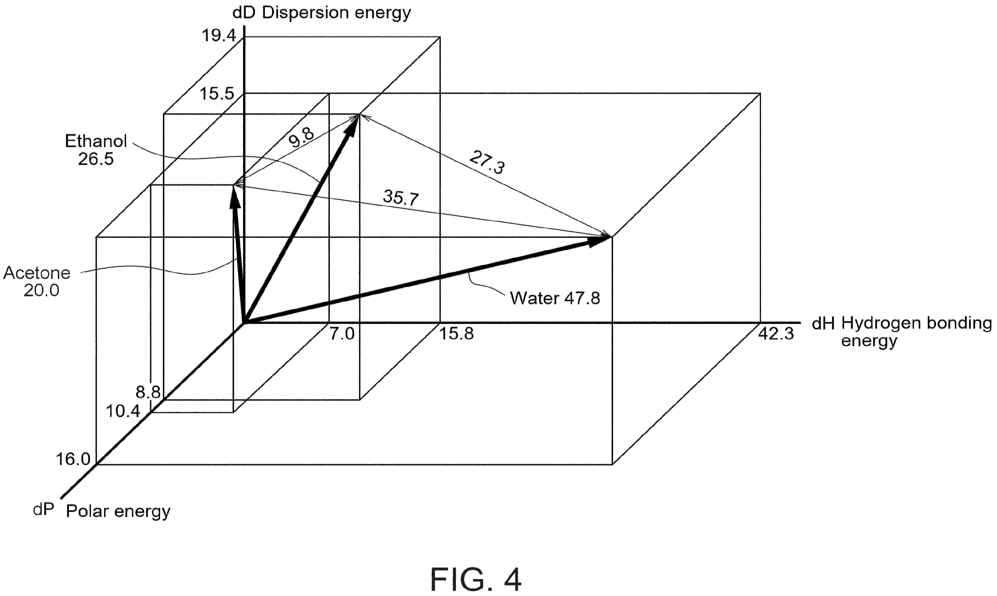

<Distance of Hansen Solubility Parameter Value (HSP distance)> The biodegradable resin, good solvent, and poor solvent that are used in the method of present invention can be selected using distance of Hansen solubility parameter values (HSP distance) as an indicator.
The rate at which the spinning solution prepared by dissolving the biodegradable resin in a good solvent is extruded from the nozzle outlet in the form of fibers and solidified in the poor solvent is determined by the distance of Hansen solubility parameter values (vector values). The distance of Hansen solubility parameter values (HSP distance) between acetone and ethanol is determined by the interaction of (i) polarization, (ii) hydrogen bonding, and (iii) dispersion between acetone and ethanol when good solvent is acetone and poor solvent is ethanol. Hansen solubility parameter value of solute polymer should be close to the solubility parameter value of the good solvent (e.g. acetone).
At 25° C, Hansen solubility parameter of ethanol is 26.5 *δ* [(MPa) ^{1/2} ]) and the Hansen solubility parameter of acetone is 20.0[(MPa )^{1/2} ]), the distance between these two is 9.8 [(MPa) ^{1/2} ] (see Figure 4).
With this distance, the spinning solution is not solidified rapidly at the tip of the nozzle, so there is no risk of clogging of the spinning solution extruded from the discharge port.
However, since the Hansen solubility parameter value of ethanol is close to that of acetone, the polymer is dissolved to a certain degree, and the spinning solution extruded in the fibrous form cannot maintain the fibrous form during the process of sinking down in ethanol and tends to be torn off during that process. In addition, since the specific gravity of ethanol is almost the same as that of acetone, the acetone that is released from the spun fiber in ethanol will remain in the vicinity of the fiber and dissolve the fiber, which is undesirable for the fiberization of the spinning solution.

When the spinning solution is prepared using acetone as a good solvent and water as a poor solvent, and the spinning solution thus prepared is extruded from the nozzle into the poor solvent in fibrous form, Hansen solubility parameter value of water is 47.8 δ [(MPa)1/2] and that of acetone is 20.0 δ [(MPa )^{1/2} ]), and HSP distance between these two is 35.7 δ [(MPa)^{1/2} ] (see Figure 4). HSP distance between water and acetone for PDLLGA is considerably larger than the HSP distance between ethanol and acetone for PDLLGA. As a result, the spinning solution extruded from the nozzle is solidified rapidly in water. If the balance between the injection speed and solidification is not matched, the spinning solution is solidified at the nozzle tip without forming a fiber, often resulting in failure of injection, making stable fiber formation difficult. On the other hand, since the specific gravity of water is considerably greater than that of acetone, acetone desorbed from the spinning solution does not stay at the bottom of the container but floats near the top. When an appropriate condition is chosen for spinning, the acetone does not cause the fibers to stick to each other, and long continuous fibers are produced. Inventors of the present invention, after careful consideration of the above behavior, continued studying the spinning process and reached a novel wet spinning process, in which the spinning solution is injected into a poor solvent having a small HSP distance from the good solvent, and then, while the solution is still not completely solidified, it is entered into a poor solvent having a larger HSP distance from the good solvent than the former so that a long and stable spinning is achieved to produce a flexible cotton-wool like material formed by independent fibers.

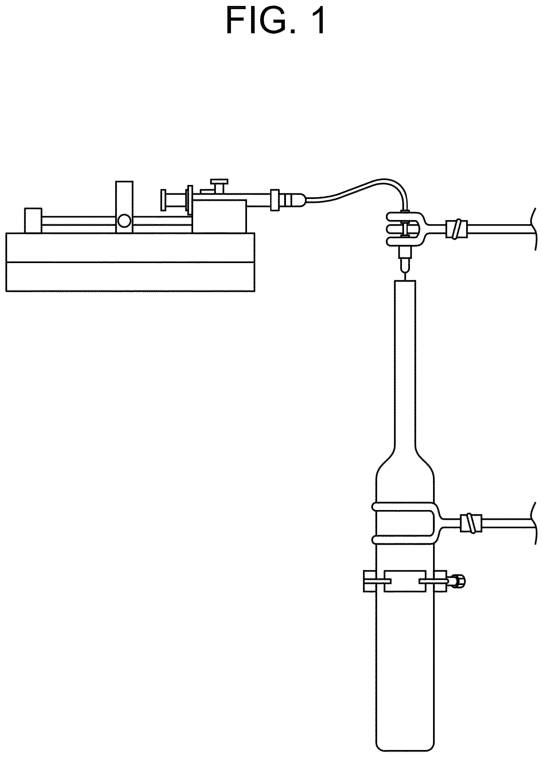

Embodiment 1 (as poor solvents, ethanol/water is used) Following materials and apparatus were used
· *β*-Tricalcium phosphate (Ca₃ (PO₄)₂ ): Taihei Chemical Industry Co. *β-*TCP-100.
   Particle size of 1.7 mm or less of the *β*-TCP-100 was ground to about 4 µm (*β*-TCP milled product).
·As PDLLGA, PDLLA:PGA (75:25) :PURASORB PDLG7507 and Corbion Purac were used.
·Ethanol: Kishida Chemical first grade, purity 99.5%.
·Acetone: Wako Pure Chemicals Reagent special grade purity 99.5+%.
·Water: Purified water
·Injection nozzle for spinning solution extrusion: Terumo injection needle 27G (inner diameter 0.2 mm, outer diameter 0.4 mm)
After filling the cylindrical collector container (see Figure 1) to a certain height with distilled water, ethanol was gently poured into the container. The specific gravity of ethanol is 789 kg/m3 (20° C), which is smaller than that of water, so water and ethanol will not mix if left to stand still.

### 1. Preparation of spinning solution

*β*-TCP and PDLLGA were mixed in 7:3 weight ratio, dissolved in acetone, and mixed overnight to prepare a spinning solution with a polymer concentration of 20%.

### 2. Spinning conditions

Injection needle (Terumo syringe needle 27G) of the wet spinning apparatus was placed vertically downward into the collector container and spinning solution was extruded at an extrusion speed of 3 ml/h into the ethanol in the collector container.

### 3. Fiberization of spinning solution

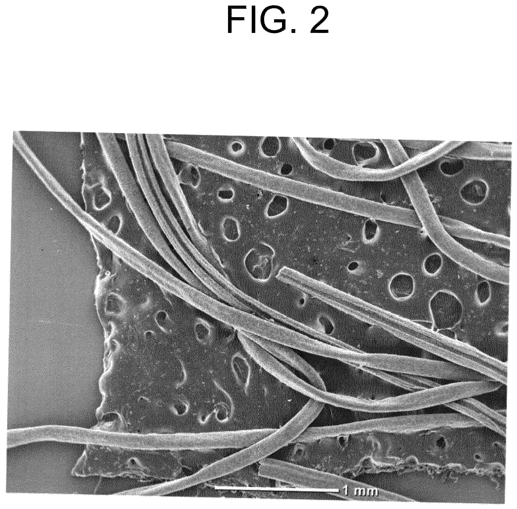

i) The spinning solution extruded from the syringe was injected into the ethanol in the collector container in fibrous form without clogging at the nozzle outlet.
ii) The fibrous spinning solution injected into ethanol was solidified on its surface, and before the acetone was completely desorbed, it was entered into the water that is filled below the ethanol in the collector container. The fibrous spinning solution passed through the water and was collected at the bottom of the collector container (see Figure 2).

### 4. Collection of cotton-wool like sample

Fibers that accumulated at the bottom of the collector container were washed with water and further held overnight to remove the solvent. The water was then removed using an absorbent sheet and dried at room temperature to obtain a cotton-wool like sample.

### 5. Properties of the obtained samples

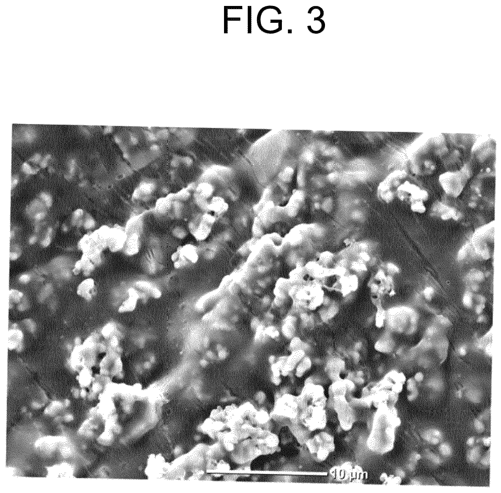

i) Diameter of the fibers was about 130 µm and the cross section of the fibers was oval in shape.
ii) *β*-TCP particles were exposed on the surface of the fiber, forming an uneven structure (see Figure 3).
Inventors checked whether the *β*-TCP particles exposed on the fiber surface were covered by a thin resin layer by immersing the sample in hydrochloric acid (if the *β*-TCP particles on the fiber surface are covered by a resin layer, the *β*-TCP particles exposed on the fiber surface of the sample are not dissolved by HCL). It was found that *β-*TCP particles that are exposed on a surface of the fibers of the sample are not covered by a resin layer.

### Embodiment 2 (As poor solvents, mixture of acetone/water and water are used)

Following materials and apparatus were used
· *β*-Tricalcium phosphate (Ca₃(PO₄)₂): Taihei Chemical Industry Co. Particle size of 1.7 mm or less was ground to about 4 µm (*β*-TCP milled product).
·As PDLLGA, PDLLA:PLGA (75:25) (PURASORB PDLG7507, Corbion Purac) was used.
·Distilled water
·Acetone: Wako Pure Chemicals Reagent special grade purity 99.5+%.
·The first poor solvent is a mixture of acetone and water at a weight ratio of 8:2 and stirred thoroughly.
·Injection nozzle for spinning solution extrusion: Terumo injection needle 27G (inner diameter 0.2 mm, outer diameter 0.4 mm) · Container of first poor solvent (mixture of acetone: water = 8:2): a cylinder with a diameter of 15 mm and a height of 50 mm was used.
· Container of second poor solvent (water): a cylindrical container with a diameter of 90 mm and a height of 300 mm was used. The first poor solvent container (cylindrical) and the second poor solvent container (cylindrical) were fixed using a clamp.

After filling the second poor solvent container with distilled water, the mixed solvent solution of acetone and water was gently poured into the first poor solvent container. Due to the difference in specific gravity, the water and mixed solvent did not mix when left to stand still.

### 1. Preparation of spinning solution

*β*-TCP and PDLLGA were mixed in 7:3 weight ratio, dissolved in acetone, and mixed overnight to prepare a spinning solution with a polymer concentration of 20%.

### 2. Spinning conditions

A syringe needle (27G diameter) was placed vertically downward and spinning solution was extruded at an extrusion rate of 3 ml/h into the first poor solvent (acetone and water).

### 3. Fiberization of spinning solution

i) The spinning solution extruded from the syringe was injected in fibrous form into the first poor solvent (acetone and water) in the collector container without clogging at the nozzle outlet.
ii) Fibrous spinning solution injected into the first poor solvent (acetone and water) was solidified on its surface. Before the acetone is completely desorbed, it was entered into the second poor solvent (water) that is filled at the bottom of the collector container, then passed through the water and deposited at the bottom of the collector container (see Figure 2).

### 4. Collection of a cotton-wool like sample

The solvent acetone in the spinning solution is replaced by the low concentration of water in the first poor solvent (a mixture of acetone and water) and solidified on the surface of the spinning solution, but it flowed out smoothly without solidifying at the nozzle tip and came into contact with the water in the second poor solvent before it was completely exchanged to the interior, and then proceeded directly to the bottom of the container and accumulated on the bottom of the container. Since the specific gravity of acetone is smaller than that of water, it does not stay at the bottom of the container, but floats near the top. As a result, even after drawing fibers for a long time, the acetone did not cause the fibers to stick to each other, and long fibers were obtained.

After wet spinning, the fibers were collected from the container and dried at room temperature on an absorbent sheet to obtain a cotton-wool like sample.

### 5. Properties of the obtained sample

i) Diameter of the fibers was about 110 µm and the cross section of the fibers was roughly circular.
ii) *β*-TCP particles were exposed on the surface of the fiber, forming an uneven shape.
The inventors checked whether the *β*-TCP particles exposed on the fiber surface were covered by the resin layer by immersing the sample in hydrochloric acid (if the *β*-TCP particles on the fiber surface are covered by the resin layer, the *β*-TCP particles will not be dissolved by hydrochloric acid). It was found that *β*-TCP particles on the surface of the sample fibers were not covered by a thin resin layer.

### Comparative experiment 1 (100% ethanol)

Experimental details: Spinning experiments were conducted under the same conditions as in Embodiment 1, but with only ethanol as the poor solvent in the collector vessel.

Result: The spinning solution filled in the syringe could be smoothly extruded from the nozzle outlet (27G) in a fiber shape, but it frequently failed to maintain its fiber shape in ethanol and was torn off.

### Comparative experiment 2 (100% water)

Experimental details: Spinning experiments were conducted under the same conditions as in Embodiment 1, but with only water as the poor solvent in the collector vessel.

Result: The spinning solution filled in the syringe frequently clogged at the nozzle outlet (27G) and could not be injected into the water.

The present invention has been described in the above examples where inorganic filler particles are contained in the spinning solution. The wet spinning method using two types of poor solvents is not limited to the production of cotto-wool like shapes, but can also be used to produce nonwoven fabrics.

## Claims

1. A method for producing biodegradable fibers containing inorganic filler particles using a wet spinning process, the method comprising:
putting a mixture of inorganic filler particles and biodegradable resin in a weight ratio of 50-80:50-20 into a mixing vessel;
charging a predetermined amount of a good solvent to the mixture in the mixing vessel so that the biodegradable resin of the mixture is dissolved by the good solvent, and stirring the dissolved mixture to prepare a spinning solution containing inorganic filler particles dispersed in the solution;
filling a syringe of a wet spinning apparatus with the spinning solution thus prepared;
excluding the spinning solution filled in the syringe through an outlet of an injection needle having a predetermined diameter at a predetermined extrusion rate in a downward direction so that the spinning solution is injected into a cylindrical collector container having a predetermined height, wherein the collector container is filled with a first poor solvent and a second poor solvent in two layers, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance;
the spinning solution extruded through the outlet of the nozzle is entered into the first poor solvent filled in the collector container in a fibrous form by its own weight, a surface of the spinning solution entered into the first poor solvent in the fibrous form is solidified by interdiffusion of desorption of the good solvent and penetration of the first poor solvent;
the spinning solution in the fibrous form having a solidified surface is then continuously entered into the second poor solvent below the first poor solvent in the collector vessel by its own weight;
the spinning solution entered into the second poor solvent in the fibrous form is further solidified to produce a solidified fiber by proceeding interdiffusion of desorption of the good solvent and penetration of the second poor solvent into an interior of the fibrous spinning solution, and the solidified fiber becomes a long continuous fiber without adhering to each other, and floating deposited at a bottom of the collector vessel; and
collecting the fibers deposited at the bottom of the collector container from the collector container and drying the collected fibers to produce a biodegradable fiber containing inorganic filler particles.

2. The method according to claim 1, wherein the biodegradable resin is PDLLGA, the good solvent is acetone, the first poor solvent is ethanol, and the second poor solvent is water.

3. The method according to claim 1, wherein the biodegradable resin is PDLLGA, the good solvent is acetone, the first poor solvent is a mixed solution of acetone and water, and the second poor solvent is water.

4. The method according to claim 1 or 2, wherein the amount of good solvent added to the mixture of inorganic filler particles and biodegradable resin is adjusted such that the inorganic filler particles are diffused uniformly in the syringe without settling down in the solution, and the spinning solution is fibered in the poor solvent by extruding the solution through the injection needle into the poor solvent.

5. The method according to claim 1 or 2, wherein an outer diameter of the fibers obtained by collecting from the collector container and drying is 80-200 *µ*m.

6. The method according to claim 1 or 2, wherein diameter of the outlet of the injection needle is 0.17-0.21 mm (27 g), and outer diameter of the fiber obtained by collecting from the collector container and drying is 100-150 *µ*m.

7. The method according to claim 1 or 2, wherein the inorganic filler particles are calcium salt particles.

8. A cotton-wool like bone regenerating material produced by using a wet spinning method, the cotton-wool like bone regenerating material is produced by the steps comprising:
putting a mixture of inorganic filler particles and biodegradable resin in a weight ratio of 50-80:50-20 into a mixing vessel;
charging a predetermined amount of a good solvent into the mixing vessel so that the biodegradable resin of the mixture is dissolved by the good solvent, and stirring the dissolved solution to prepare a spinning solution in which the inorganic filler particles are dispersed in the solution;
filling the spinning solution thus prepared in a syringe of a wet spinning apparatus;
excluding the spinning solution filled in the syringe through an outlet of an injection needle having a predetermined diameter at a predetermined extrusion rate in a vertical downward direction so that the spinning solution is injected into a cylindrical collector container having a predetermined height, wherein the collector container is filled with a first poor solvent and a second poor solvent in two layers, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance,
the spinning solution extruded through the outlet of the nozzle is entered into the first poor solvent in the collector container by its own weight in a fibrous form, a surface of the spinning solution entered into the first poor solvent in the fibrous form is solidified by interdiffusion of the desorption of the good solvent and the penetration of the first poor solvent;
the spinning solution in the fibrous form having a solidified surface is then continuously entered into the second poor solvent below the first poor solvent in the collector vessel by its own weight;
the spinning solution entered into the second poor solvent in the fibrous form is further solidified to produce a solidified fiber by allowing the interdiffusion of desorption of good solvent and penetration of second poor solvent into the interior of the spinning solution to proceed, and a long continuous fiber is produced by the solidified fibers without adhering each other and floating deposited at the bottom of the collector vessel; and
collecting the fiber deposited at the bottom of the collector container from the collector container and dried.

9. The bone regeneration material according to claim 8, wherein the biodegradable resin is PDLLGA, the good solvent is acetone, the first poor solvent is ethanol, and the second poor solvent is water.

10. The bone regeneration material according to claim 8, wherein the biodegradable resin is PDLLGA, the good solvent is acetone, the first poor solvent is a mixed solution of acetone and water, and the second poor solvent is water.

11. The bone regeneration material according to claim 8 or 9, wherein the amount of good solvent added to the mixture of inorganic filler particles and biodegradable resin is adjusted such that the inorganic filler particles are diffused uniformly in the syringe without settling down in the solution, and the spinning solution is fibered in the poor solvent by extruding the solution through the injection needle into the poor solvent.

12. A wet spinning apparatus for use in an improved wet spinning process, the apparatus comprising:
a syringe for injecting a spinning solution, the spinning solution is prepared by putting inorganic filler particles and biodegradable resin in a mixing vessel in a weight ratio of 50-80:50-20 into a mixing vessel and dissolving the biodegradable resin by using a good solvent and stirring the dissolved solution;
a nozzle connected to an end of the syringe having a discharge port at a tip end thereof; and
a cylindrical collector container having a predetermined height, the collector container comprises:
an injection section for injecting and passing the spinning solution extruded from the nozzle outlet into a first poor solvent,
a fiber solidification section for solidifying the spinning solution that has passed through the injection section into a second poor solvent, the fiber solidification section is provided adjacent below the injection section, specific gravity of the second poor solvent is larger than that of the first poor solvent, the first poor solvent has a dissolution parameter value of a first distance from that of the good solvent, and the second poor solvent has a dissolution parameter value of a second distance from that of the good solvent, the second distance is larger than the first distance,
a fiber collection section adjacent below the fiber solidification section for collecting the fiber that has passed through the fiber solidification section and floating deposited at the bottom of the collector container in a cotton-wool like form,
the fiber solidification section and the fiber collection section are separably connected each other using a clamp, and the fiber collection section can be separated from the fiber solidification section after the solidified fibers are deposited at the bottom of the collector container.

13. The wet spinning apparatus according to claim 12, wherein the biodegradable resin is PDLLGA, the good solvent is acetone, the first poor solvent is ethanol, and the second poor solvent is water.

14. The wet spinning apparatus according to claim 12, wherein the biodegradable resin is PDLLGA, the good solvent is acetone, the first poor solvent is a mixed solution of acetone and water, and the second poor solvent is water.
